# EUROPEAN PATENT SPECIFICATION

(11) **EP 0 729 931 B1**
(45) Date of publication and mention of the grant of the patent: **19.08.1998**
(21) Application number: 96102027.8
(22) Date of filing: 13.02.1996
(51) Int. Cl.: C07C 11/12, C07C 1/26

(54) **Process for the preparation of 1,5-hexadiene**
Verfahren zur Herstellung von 1,5-Hexadien
Procédé pour la fabrication du 1,5-hexadiène

(30) Priority: 03.03.1995 US 398277
(43) Date of publication of application: 04.09.1996
(73) Proprietor: DOW CORNING CORPORATION, Midland Michigan 48686-0994 (US)
(72) Inventor: Bank, Howard Marvin, Freeland, MI 48623 (US); Hayes II, Keith Quentin, Bay City, MI 48708 (US); Binh Thanh Nguyen, Midland, Michigan 48640 (US)
(74) Representative: Fleischer, Holm Herbert, Dr.

(56) References cited:
- GB-A- 1 068 712

## Description

The present invention is a one-step Grignard-type process for preparation of 1,5-hexadiene. The process comprises contacting magnesium metal with a mixture comprising allyl chloride; one to 15 moles of a dialkyl ether comprising less than seven carbon atoms, per mole of the allyl chloride; and 0.05 to less than two moles of a liquid aromatic hydrocarbon solvent per mole of the dialkyl ether; at a temperature within a range of 5°C. to 200°C. We have found that the presence of the co-solvent comprising the dialkyl ether and the liquid aromatic hydrocarbon at the described molar ratio provides for a product slurry that stirs and flows easily. These characteristics of the product slurry improve mass transfer and heat transfer during the process and allow for easier separation of the 1,5-hexadiene from the product slurry. Furthermore, the use of the co-solvent allows the process to be run as a continuous process. The process is self initiating when run within the described mole ratio of liquid aromatic hydrocarbon solvent to dialkyl ether.

The production and reactions of Grignard reagents have been the subject of books and numerous review articles. Such reviews are provided, for example, in Coates et al., **ORGANOMETALLIC COMPOUNDS**, Vol. 1, p. 76-103, (1967), Methuen and Co. LTD, London, U.K.; and in Kirk and Othmer, **ENCYCLOPEDIA OF CHEMICAL TECHNOLOGY**, Vol. 10, 721-734 (1966), The Interscience Encyclopedia, Inc., NY, NY. The structure of the Grignard reagent has not been determined with certainty. However, it is generally believed that it exists as a complex in solution and that solvent can play a critical role in complex formation. The unpredictable effect of solvent on the formation and reactivity of Grignard reagents is discussed in the above cited articles.

The preparation of 1,5-hexadiene by a process using a Grignard reagent as an intermediate is known. For example, Turk et al., **Organic Synthesis**, Vol. 27, 7-8, 1947, teach a process for preparing 1,5-hexadiene by the reaction of allyl chloride in anhydrous ether with magnesium turnings. This reaction results in the formation of a thick slurry which becomes unstirrable. The unstirrable slurry is then treated with a hydrochloric acid solution until the magnesium chloride by-product is in solution and the slurry becomes sufficiently fluid to be stirred. The ether layer is then separated and distilled to provide a yield of 1,5-hexadiene in a range of 55-65%. The basis on which the yield was calculated is not reported in that article.

The process of Turk et al. is not generally acceptable as a commercial process. The formation of the non-stirrable slurry during the reaction can cause reduced mass and heat transfer and therefore can reduce yield. Furthermore, the nature of the slurry makes it necessary to treat the slurry in an additional step with a reagent such as aqueous hydrogen chloride to allow isolation of the 1,5-hexadiene product. Typically, a major portion of the 1,5-hexadiene is trapped within the non-stirrable slurry. In addition, the non-flowable nature of the slurry does not allow for the reaction to be run as a continuous process.

Therefore, it is an objective of the present invention to provide a process for preparing 1,5-hexadiene by the reaction of allyl chloride with magnesium that results in a slurry which is flowable and easily stirred. Thus, mass transfer and heat transfer is enhanced in the reaction mixture providing for an improved yield of 1,5-hexadiene. In addition, the formation of a slurry that is flowable allows our process to be conducted as a continuous process. No additional step is necessary to solubilize the slurry to make it flowable and allow for the recovery of 1,5-hexadiene.

We have found that when allyl chloride is contacted with magnesium in the presence of a co-solvent comprising a dialkyl ether of less than seven carbon atoms and 0.05 to less than 2 moles of a liquid aromatic hydrocarbon solvent per mole of the dialkyl ether, the resulting slurry is both flowable and easily stirred. Surprisingly, yields of 1,5-hexadiene will then exceed 90 percent, based on magnesium added to the process. The flowable nature of the resulting slurry allows the process to be run as a continuous process.

U.S. Patent 3,080,324, claims that an oxygenated solvent and a liquid hydrocarbon can be used as a reaction medium in the preparation of a Grignard reagent. It does not teach that the co-solvent system is useful in subsequent reactions of the Grignard reagent.

U.S. Patent 3,801,558, discloses that advantages can be realized when the reducing agent used in preparing a magnesium-reduced catalyst is an organomagnesium Grignard reagent prepared in a hydrocarbon solvent medium containing a controlled amount of a complexing agent for the Grignard reagent such as dialkyl ether. The reported advantage is that the Grignard reagent may be more soluble in hydrocarbon solvents at ambient temperature. The patent also reports the use of the Grignard as a reducing agent for titanium trichloride in a process for making a catalyst useful in polymerizing alpha-olefins.

By "one-step" process, it is meant that it is not necessary to isolate an intermediate Grignard-type reagent in our process and then to further react this reagent to form the 1,5-hexadiene. Furthermore, it is not necessary in our process to conduct a separate solubilization step on the resulting product slurry to facilitate recovery of the 1,5-hexadiene.

Our process comprises reacting magnesium metal with allyl chloride in a co-solvent mixture. The method of making the magnesium metal and the physical form of the magnesium metal can be any of those known in the art. The magnesium metal can be in the form of powder, chips and shavings. A preferred form of magnesium metal is shavings.

Contact of the magnesium metal with the allyl chloride is effected in standard type reactors suitable for running Grignard-type reactions. The reactor can be of a batch, semi-batch or continuous type. A preferred reactor is a continuous reactor. The environment in which the present process is run should be inert. Therefore, in a preferred process, the magnesium metal is contacted with the allyl chloride in a reactor which has been purged and blanketed with an inert gas such as, for example, nitrogen or argon.

Typically, the magnesium metal is added to the reactor containing the co-solvent mixture and allyl chloride in an additional co-solvent mixture. The product mixture is then fed to the reactor at a controlled rate. The mole ratio of magnesium to allyl chloride fed to the reactor is not critical and can be varied within wide limits. For a batch process, it is preferred that the mole ratio of magnesium to allyl chloride will provide allyl chloride in sufficient excess to ensure essentially total conversion of the magnesium to magnesium salts. When our process is conducted as a continuous process, the magnesium metal is typically present in excess in relation to the allyl chloride fed to the reactor. In such a case, the rate of feed of allyl chloride to the reactor is controlled to assure acceptable levels of conversion of the allyl chloride to 1,5-hexadiene and to minimize the presence of unreacted allyl magnesium chloride complexes. The allyl chloride feed may even be split, with a portion being added after the magnesium bed to insure complete reaction of the allyl magnesium chloride. Any excess allyl chloride is then conveniently recovered and recycled to the process.

The claimed process is conducted in the presence of a co-solvent mixture comprising a dialkyl ether of less than seven carbon atoms and a liquid aromatic hydrocarbon solvent. The dialkyl ether can be dimethyl ether, diethyl ether, ethylmethyl ether and n-butylmethyl ether. The preferred ether is diethyl ether. One to fifteen moles of the dialkyl ether are added to the present process per mole of allyl chloride. Preferred is when three to ten moles of dialkyl ether are added. Even more preferred is when two to five moles of dialkyl ether are added.

The liquid aromatic hydrocarbon solvent, can be any aromatic hydrocarbon solvent that is a liquid under process conditions. The liquid aromatic hydrocarbon solvent can be, for example, toluene, xylene and benzene. A preferred liquid aromatic hydrocarbon solvent is toluene.

The mole ratio of the dialkyl ether to the liquid aromatic hydrocarbon is critical to the present process. Our method requires the presence of 0.05 to less than two moles of the liquid aromatic hydrocarbon solvent per mole of the dialkyl ether. At a ratio of two moles or greater of solvent per mole of dialkyl ether, our reaction process does not readily initiate. At a mole ratio of less than 0.05 of solvent per mole of dialkyl ether, the resulting slurry has a paste-like consistency and may require solubilization for efficient recovery of the 1,5-hexadiene. It is preferred that the mole ratio of liquid aromatic hydrocarbon solvent to dialkyl ether be within a range of 0.2 to 1.5.

The present process is run at a temperature within a range of 5°C. to 200°C. It is preferred that the process be run at a temperature between 30°C. and 170°C. The pressure at which our process is run is not critical and it can be ambient to 1.48 MPa (215 psi). A preferred gauge pressure is within a range of from 0 to 0.862 MPa (0 to 125 psi).

The product of the present process is 1,5-hexadiene in a stirrable slurry. In addition to 1,5-hexadiene, this slurry may comprise dialkyl ether, liquid aromatic hydrocarbon solvent, magnesium chloride salts, unreacted magnesium and other solids. The 1,5-hexadiene is further isolated by separating the slurry into a liquid fraction containing 1,5-hexadiene and a solid fraction containing magnesium chloride salts, unreacted magnesium and other solids. Such separation is effected by standard means for separating liquids from solids such as settling or filtration. The liquid portion comprising the 1,5-hexadiene in co-solvents can be further separated by, for example, distillation to separate the co-solvents from the 1,5-hexadiene. The co-solvents may also be recycled to the process.

### Example 1

A series of runs were conducted to evaluate the effect of toluene as a co-solvent in a process for making 1,5-hexadiene. The process was run in a reactor comprising a one liter flask equipped with a reflux condenser and a dry-ice condenser located on top of the reflux condenser. The flask was also equipped with a heating mantle and a mechanism for stirring. Magnesium metal shavings, anhydrous diethyl ether, toluene and n-octane as an internal standard, were added to the reactor. The reactor was blanketed with nitrogen gas and the contents heated to reflux. A mixture comprising allyl chloride, diethyl ether and toluene was added to the reactor dropwise at a rate sufficient to sustain the temperature reported in Table 1. The allyl chloride was added to the reactor at 25 percent stoichiometric excess in relation to the magnesium for runs 1-2 and at 7 percent stoichiometric excess for runs 3-4B. The mole ratio of diethyl ether, toluene and allyl chloride added to the reactor for each run is provided in Table 1.

In run 4A, with a diethyl ether (Et₂O), toluene and allyl chloride (allyl-Cl) ratio of 2:4:1, the reaction did not initiate. In run 4B, additional diethyl ether was added to the mixture of run 4A to provide for a mole ratio of diethyl ether, toluene and allyl chloride of 4:4:1. At the times reported in Table 1, a sample of the reaction mixtures were taken and analyzed by gas chromatography using a flame ionization detector (GC-FID). The percent yield of 1,5-hexadiene is reported in Table 1 and is based upon the amount of magnesium added to the reactor. At the times indicated in Table 1, the viscosity of the resultant slurry was assessed. The viscosity of the slurry was assessed by the ease with which the slurry could be stirred, with a "Very Low" viscosity being freely stirrable and comparable to the viscosity of the mixture originally formed in the reactor and a "Very High" viscosity being a non-stirrable material of paste-like consistency.

**Table 1**

| Toluene as Co-Solvent in a Process For Preparation of 1,5-Hexadiene | | | | | | | |
|---|---|---|---|---|---|---|---|
| Mole Ratio | | | | | | | |
| Run No. | Et₂O | Toluene | Allyl-Cl | Temp.(°C) | Time(h) | % Yield Hexadiene | Slurry Viscosity |
| 1 | 10 | 4 | 1 | 44 | 5.9 | 88 | Low |
| 2 | 6 | 2 | 1 | 32 | 3.7 | 90 | Low |
| 3 | 3 | 3 | 1 | 32 | 4.2 | 90 | Very Low |
| 4A | 2 | 4 | 1 | 32 | 1.0 | 0 | - |
| 4B | 4 | 4 | 1 | 32 | 5.2 | 84 | Very Low |

### Example 2 (Comparative runs)

A series of runs were conducted in the absence of toluene as a co-solvent in a process for making 1,5-hexadiene. The runs were conducted in a reactor similar to that described in Example 1. Magnesium metal shavings, anhydrous diethyl ether and n-octane as internal standard, were added to the reactor. A nitrogen blanket was formed in the reactor and the content heated to reflux. For run 6, the reactor content was initially cooled to 15°C. and the temperature allowed to rise to reflux during addition of the allyl chloride. A few crystals of iodine were added to the reactor for runs 6 and 7 to serve as an initiator. A mixture comprising allyl chloride and diethyl ether was added to the reactor dropwise at a rate sufficient to sustain the temperature reported in Table 2. The allyl chloride was added to the reactor at 25 percent stoichiometric excess in relation to the magnesium. The mole ratio of diethyl ether to allyl chloride added to the reactor is also provided in Table 2. At the times reported in Table 2, a sample of the reaction mixtures were taken and analyzed by GC-FID. The percent yield of 1,5-hexadiene is reported in Table 2 and is based upon the amount of magnesium added to the reactor. At the times reported in Table 2, the viscosity of the slurry present in the reactor was assessed and is reported in Table 2.

**Table 2**

| Single Solvent Process For Preparation of 1,5-Hexadiene | | | | | | |
|---|---|---|---|---|---|---|
| Mole Ratio | | | | | | |
| Run No. | Et₂O | Allyl-Cl | Temp.(°C) | Time(h) | % Yield Hexadiene | Slurry Viscosity |
| 5 | 10 | 1 | 32 | 2.2 | 96 | High |
| 6 | 6 | 1 | 32 | 5.7 | 81 | Very High |
| 7 | 8 | 1 | 32 | 2.3 | 64 | Very High |
| 8 | 8 | 1 | 32 | 3.7 | 67 | Very High |

### Example 3

A run was conducted to evaluate the effect of toluene as a co-solvent in a process for preparation of 1,5-hexadiene at 150°C. The evaluation was conducted in a sealed glass tube. Magnesium metal (0.13 g) and 0.3 mL of a mixture comprising allyl chloride, toluene and diethyl ether at a mole ratio of 1:3:3 was placed in the tube. The tube contents were quickly frozen in an IPA/dry ice bath and then the tube was heat sealed. The sealed tube was placed in a tube furnace preheated to 150°C. After approximately two minutes, the tube was removed from the tube furnace and cooled in the IPA/dry ice bath. The content of the tube was analyzed by GC-FID. The analysis indicated 100 percent conversion of allyl chloride to 1,5-hexadiene.

### Example 4 (Comparative run)

The ability to run a co-solvent process for preparation of 1,5-hexadiene in the presence of a co-solvent comprising n-dibutyl ether and toluene was evaluated. The evaluation was conducted in a reactor similar to that described in Example 1. Magnesium metal shavings, anhydrous n-dibutyl ether, toluene and n-octane as an internal standard, were added to the reactor. A nitrogen blanket was formed in the reactor and the content heated to reflux. A mixture comprising allyl chloride, n-dibutyl ether and toluene was slowly added to the reactor and the mixture externally heated to raise the temperature to 100°C. The allyl chloride was added to the reactor at 25 percent stoichiometric excess in relation to the magnesium. The mole ratio of n-dibutyl ether, toluene and allyl chloride added to the reactor was 3:3:1. The mixture was held at 100°C. for six hours. At the end of six hours, a sample was taken from the reactor and analyzed by GC-FID. The yield of 1,5-hexadiene was six percent, based on the amount of magnesium added to the reactor.

### Example 5

A run was conducted to evaluate the effect of toluene as a co-solvent in a continuous process for preparation of 1,5-hexadiene. The reactor was a (1000 gallon) reactor containing a stirred bed of magnesium. Diethyl ether, toluene and allyl chloride at a mole ratio of 5:3:1 were passed into the bottom of the reactor at a rate providing for a residence time in the reactor of three hours. Magnesium shavings were periodically added to the reactor to keep magnesium in excess. The temperature in the magnesium bed was maintained at 106°C., while the bulk of the content of the reactor above the magnesium was maintained at 51°C. The gauge pressure in the reactor was 0.758 MPa (110 psi). Reaction mixture continuously flowed from the top of the reactor to a second reactor maintained at 85°C. and a gauge pressure of 0.193 MPa (28 psi), where excess allyl chloride in toluene was added to insure complete reaction of the allyl magnesium chloride complex. The product exiting the second reactor was analyzed by GLC-FID and found to consist of 24.9 area percent diethyl ether, 61.1 area percent toluene, 10.4 area percent 1,5-hexadiene and 2.2 area percent allyl chloride. A test for active Grignard reagent was negative indicating all the allyl magnesium chloride had been consumed. During the process, the slurry in both reactors remained a low viscosity solution that flowed readily and was easily stirred. The yield of 1,5-hexadiene, based on allyl chloride consumed, was within a range of 80 to 90 percent.

## Claims

1. A one-step process for preparation of 1,5-hexadiene, the process comprising contacting magnesium metal with a mixture comprising allyl chloride; one to 15 moles of a dialkyl ether comprising less than seven carbon atoms, per mole of the allyl chloride; and 0.05 to less than two moles of a liquid aromatic hydrocarbon solvent per mole of the dialkyl ether; at a temperature within a range of 5°C to 200°C and a pressure within a range of ambient pressure to 1.48 MPa.

2. A process according to claim 1 where the magnesium metal is contacted with the mixture by directing a continuous flow of the mixture through a bed of magnesium metal.

3. A process according to claim 1 where the magnesium metal is contacted with the mixture in an inert environment.

4. A process according to any of claims 1-3 where the magnesium metal is in the form of shavings.

5. A process according to claim 1 where the liquid aromatic hydrocarbon solvent is selected from a group consisting of toluene, xylene and benzene.

6. A process according to claim 1 where the process is run as a continuous process, the dialkyl ether is diethyl ether, the mixture comprises three to ten moles of diethyl ether per mole of allyl chloride, the liquid aromatic hydrocarbon solvent is toluene, the mole ratio of toluene to diethyl ether in the mixture is within a range of 0.2 to 1.5, the temperature is within a range of 30°C to 170°C and the magnesium metal is contacted with the mixture at a gauge pressure within a range from 0 to 0.862 MPa (0 to 125 psi).

7. A process according to claim 6 where the magnesium metal is contacted with the mixture in an inert environment comprising nitrogen gas.

## Patentansprüche

1. Einstufiges Verfahren zur Herstellung von 1,5-Hexadien, wobei das Verfahren umfaßt Inkontaktbringen von Magnesiummetall mit einer Mischung, die Allylchlorid, 1 bis 15 Mole eines Dialkylethers, der weniger als 7 Kohlenstoffatome enthält, pro Mol des Allylchlorids und 0,05 bis weniger als 2 Mole eines flüssigen aromatischen Kohlenwasserstofflösungsmittels pro Mol des Dialkylethers enthält, bei einer Temperatur innerhalb eines Bereichs von 5°C bis 200°C und einem Druck innerhalb eines Bereichs von Umgebungsdruck bis 1,48 MPa umfaßt.

2. Verfahren nach Anspruch 1, wobei das Magnesiummetall mit der Mischung durch Hindurchleiten eines kontinuierlichen Stroms der Mischung durch ein Bett aus Magnesiummetall in Kontakt gebracht wird.

3. Verfahren nach Anspruch 1, wobei das Magnesiummetall mit der Mischung in einer inerten Umgebung in Kontakt gebracht wird.

4. Verfahren nach einem der Ansprüche 1 bis 3, wobei das Magnesiummetall in Form von Spänen vorliegt.

5. Verfahren nach Anspruch 1, wobei das flüssige aromatische Kohlenwasserstofflösungsmittel aus der Gruppe bestehend aus Toluol, Xylol und Benzol ausgewählt ist.

6. Verfahren nach Anspruch 1, wobei das Verfahren als kontinuierliches Verfahren durchgeführt wird, der Dialkylether Diethylether ist, die Mischung 3 bis 10 Mole Diethylether pro Mol Allylchlorid enthält, das flüssige aromatische Kohlenwasserstofflösungsmittel Toluol ist, das Molverhältnis von Toluol zu Diethylether in der Mischung innerhalb eines Bereichs von 0,2 bis 1,5 liegt, die Temperatur innerhalb eines Bereichs von 30°C bis 170°C liegt und das Magnesiummetall mit der Mischung bei einem Überdruck in einem Bereich von 0 bis 0,862 MPa (0 bis 125 psi) in Kontakt gebracht wird.

7. Verfahren nach Anspruch 6, wobei das Magnesiummetall mit der Mischung in einer inerten Umgebung, die Stickstoffgas enthält, in Kontakt gebracht wird.

## Revendications

1. Procédé en une étape pour la préparation de 1,5hexadiène, le procédé comprenant la mise en contact de magnésium métallique avec un mélange comprenant du chlorure d'allyle ; une à 15 moles d'un éther dialkylique comprenant moins de sept atomes de carbone, par mole du chlorure d'allyle ; et 0,05 à moins de deux moles d'un solvant hydrocarboné aromatique liquide par mole de l'éther dialkylique ; à une température dans la gamme de 5 °C à 200 °C et une pression dans la gamme de la pression ambiante à 1,48 MPa.

2. Procédé selon la revendication 1, dans lequel le magnésium métallique est mis en contact avec le mélange en envoyant un flux continu du mélange à travers un lit de magnésium métallique.

3. Procédé selon la revendication 1, dans lequel le magnésium métallique est mis en contact avec le mélange dans un environnement inerte.

4. Procédé selon l'une quelconque des revendications 1 à 3, dans lequel le magnésium métallique est sous la forme de copeaux.

5. Procédé selon la revendication 1, dans lequel le solvant hydrocarboné aromatique liquide est choisi dans le groupe constitué par le toluène, le xylène et le benzène.

6. Procédé selon la revendication 1, dans lequel le procédé est mis en oeuvre sous forme d'un procédé continu, l'éther dialkylique est l'éther diéthylique, le mélange comprend trois à dix moles d'éther diéthylique par mole de chlorure d'allyle, le solvant hydrocarboné aromatique liquide est le toluène, le rapport molaire du toluène à l'éther diéthylique dans le mélange est dans la gamme de 0,2 à 1,5, la température est dans la gamme de 30 °C à 170 °C et le magnésium métallique est mis en contact avec le mélange à une pression manométrique dans la gamme de 0 à 0,862 MPa (0 à 125 psi).

7. Procédé selon la revendication 6, dans lequel le magnésium métallique est mis en contact avec le mélange dans un environnement inerte comprenant de l'azote gazeux.
